# EUROPÄISCHE PATENTSCHRIFT

(11) **EP 2 152 462 B1**
(45) Veröffentlichungstag und Bekanntmachung des Hinweises auf die Patenterteilung: **24.05.2017**
(21) Anmeldenummer: 08758177.3
(22) Anmeldetag: 07.06.2008
(51) Int. Cl.: B23K 26/03, B23K 26/40, B23K 26/06, A61F 9/008

(54) **VERFAHREN ZUR LASERBEARBEITUNG TRANSPARENTER MATERIALIEN**
METHOD FOR LASER MACHINING TRANSPARENT MATERIALS
PROCÉDÉ D'USINAGE LASER DE MATÉRIAUX TRANSPARENTS

(30) Priorität: 14.06.2007 DE 102007028042
(43) Veröffentlichungstag der Anmeldung: 17.02.2010
(73) Patentinhaber: Universität zu Lübeck, 23538 Lübeck (DE)
(72) Erfinder: VOGEL, Alfred, 23568 Lübeck (DE); LINZ, Norbert, 23562 Lübeck (DE); FREIDANK, Sebastian, 23568 Lübeck (DE)
(74) Vertreter: Appelt, Christian W.
(86) Internationale Anmeldenummer: PCT/DE2008/000955
(87) Internationale Veröffentlichungsnummer: WO 2008/151616

(56) Entgegenhaltungen:
- DE-A1- 10 323 422
- VOGEL A ET AL: "Mechanisms of femtosecond laser nanosurgery of cells and tissues" APPLIED PHYSICS B ; LASERS AND OPTICS, SPRINGER, BERLIN, DE, Bd. 81, Nr. 8, 1. Dezember 2005 (2005-12-01), Seiten 1015-1047, XP019337596 ISSN: 1432-0649
- RAU KAUSTUBH R ET AL: "Pulsed laser microbeam-induced cell lysis: Time-resolved imaging and analysis of hydrodynamic effects" BIOPHYSICAL JOURNAL, NEW YORK, US, US, Bd. 91, Nr. 1, 1. Juli 2006 (2006-07-01), Seiten 317-329, XP002480409 ISSN: 0006-3495
- COLOMBELLI JULIEN ET AL: "Ultraviolet diffraction limited nanosurgery of live biological tissues", REVIEW OF SCIENTIFIC INSTRUMENTS, AIP, MELVILLE, NY, US, vol. 75, no. 2, 1 February 2004 (2004-02-01), pages 472-478, XP012071303, ISSN: 0034-6748, DOI: 10.1063/1.1641163

## Beschreibung

Die Erfindung betrifft ein Verfahren zur Laserbearbeitung transparenter Materialien, insbesondere zur Erzeugung präzise lokalisierter Materialveränderungen im Inneren eines transparenten Körpers.

Die Erzeugung sehr feiner Effekte bei der Lasermaterialbearbeitung setzt die lokalisierte Deponierung sehr geringer Energiemengen voraus. Wenn die Energiedeponierung durch lineare Absorption (1-Photonen-Absorption) erfolgt, verlangt die gewünschte hohe Präzision der Materialbearbeitung eine geringe optische Eindringtiefe der Laserstrahlung sowie eine hinreichend kurze Laserpulsdauer, um Wärmediffusion während der Laserpulsdauer zu vermeiden.

In transparenten Materialien wie z.B. Glas, Quarz, Wasser, unpigmentiertem Körpergewebe oder Zellen kann eine lokalisierte Energiedeponierung nur durch nichtlineare Absorption erfolgen, d.h. durch Mehrphotonenprozesse in der Form von Multiphotonenionisation und Lawinenionisation, die zur Ausbildung eines Plasmas führen (quasifreie Ladungsträger im Material bestehend aus einer Mischung von Elektronen und Ionen). Da das Auftreten der Mehrphotonenprozesse nichtlinear von der Laserlichtintensität abhängt, spricht man von "nichtlinearer Absorption". Und da die Plasmabildungsrate oberhalb einer Schwelle, die von Material und Laserparametern abhängt, extrem stark zunimmt, wird der Plasmabildungsprozess in diesem Parameterbereich auch "optischer Durchbruch" genannt.

Eine hohe Präzision der Materialbearbeitung durch nichtlineare Absorption erfordert, dass räumlich lokalisiert reproduzierbar geringe Energiemengen in das Material eingetragen (deponiert) werden können. Die gute räumliche Lokalisation wird in erster Linie durch Fokussierung der Laserpulse mittels aberrationsfreier Optiken hoher numerischer Apertur erreicht.

Im Stand der Technik wird davon ausgegangen, dass geringer Energieeintrag bei hoher Reproduzierbarkeit erheblich besser durch ultrakurze Laserpulse mit einer Dauer im Bereich von einigen Femtosekunden bis wenigen Pikosekunden erfolgen kann als mit längeren Pulsen (Nanosekunden oder sogar Mikrosekunden). Hierfür gibt es vor allem drei Gründe:
1. Die experimentell detektierbare Schwelle für den optischen Durchbruch wird - vor allem bei Pulsdauern im Nanosekundenbereich - üblicherweise mit der Beobachtung eines Plasmaleuchtens gleichgesetzt.
2. Die Energieschwelle für die Bildung leuchtender Plasmen nimmt mit sinkender Pulsdauer stark ab - etwa um einen Faktor 200, wenn die Pulsdauer von 10 ns auf 100 fs verringert wird. Das Auftreten eines leuchtenden Plasmas ist daher bei Nanosekundenpulsen mit einer viel höheren Energiedichte im Plasma und dadurch mit viel stärkeren mechanischen Lasereffekten und Nebenwirkungen der eigentlichen Materialabtragung verknüpft.
3. Nahe der Schwelle für den optischen Durchbruch weisen die von üblichen Nanosekundenpulsen erzeugten Lasereffekte eine viel größere Streubreite auf als die Femtosekundeneffekte.

Dies hat zu der Auffassung geführt, der optische Durchbruch mit Nanosekundenpulsen sei generell "statistischer Natur", während der Femtosekunden-Durchbruch "deterministisch" sei und daher besser für reproduzierbare Materialbearbeitung geeignet. Überdies ist die Aussage berechtigt, das Auftreten von Plasmaleuchten bei Nanosekundenpuls-Laserbearbeitung stehe einer präzisen Lokalisierung der Lasereffekte prinzipiell entgegen.

Bedauerlicherweise sind Pulslasersysteme mit Pulsdauern unter 100 ps komplexe Systeme mit entsprechend hohen Anschaffungskosten. Deshalb wird immer wieder nach Kompromissen gesucht, die den oben begründeten Wunsch nach Kurzzeitpulsen mit erschwinglichen Geräten verbinden.

Dies ist z.B. in der Arbeit von Colombelli et al., "Ultraviolet diffraction limited nanosurgery of live biological tissues", Rev. Sci. Instrum., Vol. 75, 472-478 (2004) (=XP-A-12071303), der Fall, wo ein UV-Chip-Laser (frequenzverdreifachter Nd:YAG) mit Pulsdauer 500 ps als Laserskalpell u. a. für Einzelzellen erfolgreich verwendet wird. Solche Mikrochip-Laser sind relativ günstig, arbeiten aber üblicherweise mit Nanosekundenpulsen und können Pulsdauern von einigen 100 ps prinzipiell nicht unterschreiten.

Präzise Materialbearbeitung mit Nanosekundenpulsen in transparenten Medien ist nach der Lehre der DE 198 55 623 C1 zur Ausführung der bekannten Laserinnengravur von Glas möglich; dort werden sogar Pulsdauern von 100 ns verwendet. Allerdings besagt die Patentschrift ausdrücklich, dass man Wellenlängen außerhalb des Plateaubereichs des Transmissionsgrades verwenden soll, also solche, für die das Material eben gerade nicht optimal transparent ist. Die Bedeutung des dadurch bedingten Auftretens linearer Absorption wird von den Autoren hervorgehoben, aber nicht näher erläutert.

Ein Verfahren zur Detektion kleinster transienter Materialveränderungen wird in einer anderen Patentanmeldung der Anmelderin (DE 10 2007 003 600.2) vorgeschlagen. Es handelt sich um ein Verfahren zur möglichst schonenden Laserperforation von Zellmembranen, bei dem das Pulslaserlicht im Fokus Blasen in unmittelbarer Nachbarschaft der Zelle erzeugt. Die Größenbestimmung der Blasen kann über eine Messung der Oszillationszeit erfolgen, indem man das Zeitverhalten der Lichtintensitätsänderung eines Probelaserstrahls (cw-Laser, bevorzugt andere Wellenlänge) erfasst, der während der Bearbeitung mit gepulster Strahlung (fsbis ps-Pulse) durch den Laserfokus geführt wird. Blasengrößen bis hinab zu 150 nm sind so detektiert worden.

Es ist nun die Aufgabe der Erfindung, ein Verfahren zur Materialbearbeitung transparenter Medien mittels nichtlinearer Absorption aufzuzeigen, das auch bei Benutzung von Pulslasern mit Pulsdauern im Nanosekundenbereich auf genau lokalisierte Materialveränderungen führt.

Die Aufgabe wird gelöst mit dem Verfahren gemäß Anspruch 1. Die Unteransprüche geben vorteilhafte Ausgestaltungen an.

Den Ausgangspunkt der Erfindung bildet die Entdeckung, dass es bei der Applikation von Nanosekundenpulsen auf transparentes Material einen Strahlungsstärkenbereich gibt, in dem bereits Materialveränderung stattfindet ohne dass es zu Plasmaleuchten kommt. Man bewegt sich dabei unter der bekannten Schwelle des optischen Durchbruchs, mit der bei Pulsdauern im Nanosekundenbereich weit reichende Zerstörungen auch außerhalb des Laserfokus assoziiert sind.

Der entdeckte und nun erfindungsgemäß zu nutzende Strahlungsstärkenbereich wird je nach Material (z.B. Glas oder Wasser) beidseitig durch messbare Schwellenwerte begrenzt. Die Energieschwellen hängen dabei klar von der benutzten Pulsdauer und Laserwellenlänge ab, die untere Intensitätsschwelle für den Bereich der Laserbearbeitung variiert jedoch nur recht schwach mit der Länge der Pulse und der Wellenlänge.

Der höhere Schwellenwert wird jeweils durch das Einsetzen des Plasmaleuchtens angezeigt (i.F. Plasmaleuchtschwelle). Es kommt nach Einsetzen des Plasmaleuchtens zu Lawinenionisation und thermischer Ionisation in einem solchen Umfang, dass sich Materialschäden nicht mehr auf den Laserfokus beschränken.

Der niedrigere Schwellenwert ist hingegen jener, bei denen erste Materialeffekte detektierbar sind, insbesondere das Material einen lokalen Phasenübergang zeigt. Ein Beispiel hierfür ist der Beginn der Bildung von Blasen in Flüssigkeiten (oder Hohlräumen in Festkörpern).

Zur Klarstellung sei an dieser Stelle bereits bemerkt, dass alle Materialveränderungen in der vorliegenden Beschreibung mit Plasmabildung verbunden sind. Es ist jedoch zu unterscheiden zwischen dem leuchtenden Plasma (s. u. high-density Plasma) und jenem, welches bereits unterhalb der Plasmaleuchtschwelle - aber eben gerade oberhalb des niedrigen Schwellenwertes - entsteht und auf das Material einwirkt, aber keine weit reichenden Effekte erzeugt (s. u. low-density Plasma).

Mit der im Stand der Technik zitierten Arbeit der Anmelderin gelingt es, den niedrigen Schwellenwert (i. F. Bearbeitungsschwelle) konkret z.B. in Wasser zu erfassen. Dabei zeigt sich die allgemeine Regel, dass Bearbeitungs- und Plasmaleuchtschwelle umso weiter voneinander entfernt sind, desto kürzer die verwendete Wellenlänge gewählt wird und desto länger die Laserpulsdauer ist.

Allerdings fallen beide Schwellwerte offenbar stets zusammen für Pulsdauern unter 300 ps.

Wie eingangs gesagt, herrscht die Auffassung vor, die präzise Laserbearbeitung transparenter Materialien sei nur mit Ultrakurzpulslasern (< 100 ps Pulsdauer) effektiv durchführbar. Von daher überrascht es nicht, dass die Existenz eben jener getrennten Schwellenwerte mit dem dazwischen liegenden, erfindungsgemäß zur präzisen Materialbearbeitung nutzbaren Strahlungsstärkenbereich bislang verborgen blieb.

Um eine Lehre zum technischen Handeln zu formulieren, bleibt noch das Vorurteil auszuräumen, Nanosekundenpulslaser würden grundsätzlich zu statistisch streuenden Lasereffekten führen.

Dies soll in der folgenden näheren Erläuterung der Erfindung anhand der Figuren geschehen, in denen:

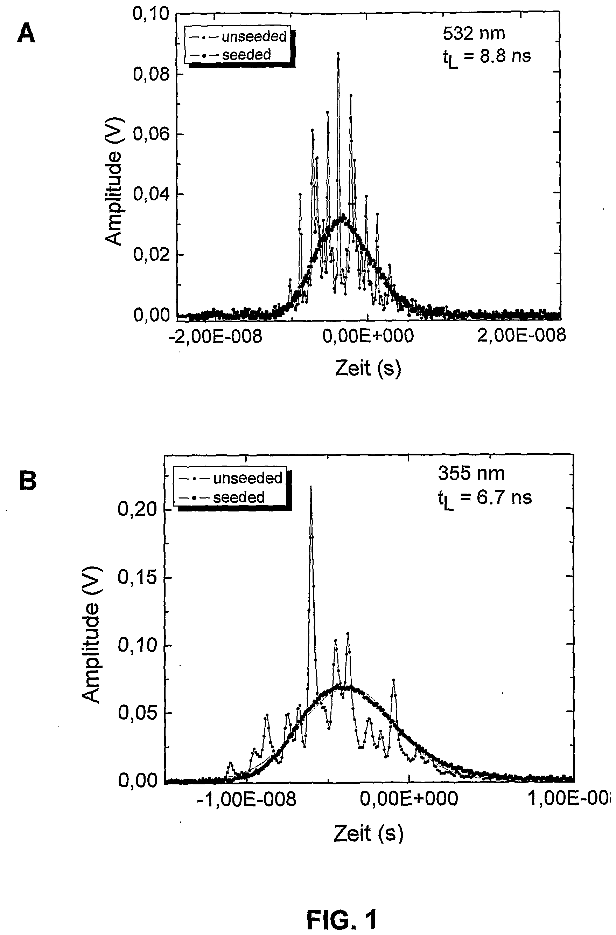

- Fig. 1: den zeitlichen Verlauf der Laserintensität bei Pulsen gleicher Energie aus einem geseedeten und einem ungeseedeten (regulären) Nd:YAG Laser zeigt; es werden in Fig. 1A die Pulsformen der frequenzverdoppelten (λ = 532 nm) und in Fig. 1B die Pulsformen der frequenzverdreifachten (λ = 355 nm) Laserstrahlung gezeigt.

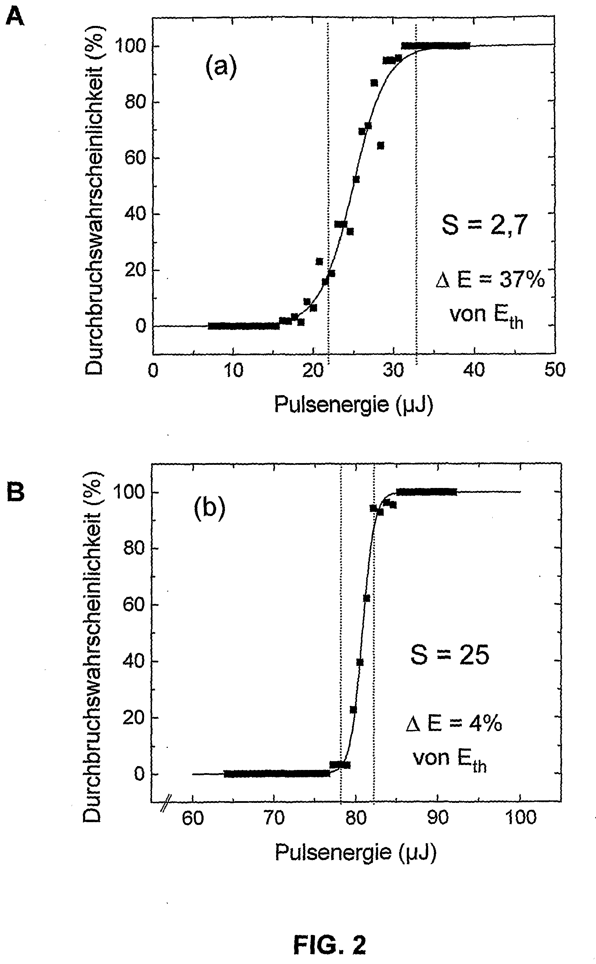

- Fig. 2: die Abhängigkeit der Durchbruchswahrscheinlichkeit von der Laserpulsenergie für den optischen Durchbruch in Wasser mit (A) ungeseedeten und (B) geseedeten Laserpulsen (Fokussierung mit NA = 0.8, λ = 1064 nm) zeigt, wobei die Schwelle für den optischen Durchbruch durch den Energiewert *E*ₜₕ definiert ist, bei dem die Durchbruchswahrscheinlichkeit 50% beträgt, und die Schwellenschärfe *S* dem Verhältnis von *E*ₜₕ zum Energieintervall *ΔE* entspricht, in dem die Durchbruchswahrscheinlichkeit von 10% auf 90% ansteigt. Ein hoher Wert von S entspricht einer scharfen, in hohem Maße reproduzierbaren Schwelle. In der Abbildung sind die Messdaten für die Bearbeitungsschwelle dargestellt.

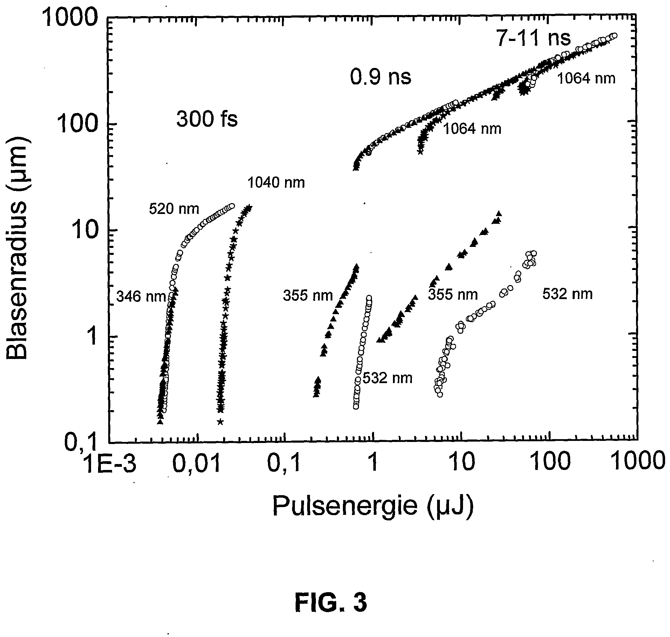

- Fig. 3: den Blasenradius als Funktion der Laserpulsenergie für Laserpulse unterschiedlicher Dauer und zeitlich glatter Pulsform (NA = 0.8) zeigt. Die für die Messung verwendeten Lasersysteme waren ein Yb:Glas Lasersystem mit Oszillator und regenerativem Verstärker für die 300-fs Pulse, ein Nd:YAG Mikrochiplaser für die 0.9-ns Laserpulse und ein geseedeter Nd:YAG Laser für die Pulse mit 7-11 ns Dauer.

Laserpulse aus "normalen" ns-Laseroszillatoren weisen aufgrund der Überlagerung vieler Longitudinalmoden (,longitudinal mode beating') ausgeprägte Intensitätsspitzen auf, deren Struktur und Amplitude von Puls zu Puls wechselt. Dieser Sachverhalt ist zwar seit langem prinzipiell bekannt, das Ausmaß der Intensitätsüberhöhungen gegenüber der zeitlich oder über viele Pulse gemittelten Pulsform erschließt sich aber erst durch Messungen mit zeitlich hoch auflösenden Fotodetektoren und Oszilloskopen.

Die Messkurven in Fig. 1 wurden mit einem Fotodetektor mit <100 ps Anstiegszeit und einem Oszilloskop mit 3 GHz Bandbreite aufgezeichnet. Aus den Messkurven ist ersichtlich, dass der angeblich "statistische Charakter" des optischen Durchbruchs mit Nanosekundenpulsen weitgehend auf statistische Schwankungen der verwendeten Laserpulse zurückzuführen ist. Mit zeitlich "glatten", z.B. gaußförmigen, Pulsen ist ein erheblich besser reproduzierbares Verhalten zu erzielen.

Gaußförmige Laserpulse werden emittiert, wenn in dem Laserresonator nur ein Longitudinal-mode anschwingen kann. Dies kann durch Betrieb nahe an der Laserschwelle in Verbindung mit einem kurzen Laserresonator und/oder mit einem Etalon im Resonator erreicht werden (die letzten beiden Maßnahmen führen zu großem Frequenzabstand der Longitudinalmoden). Mikrochiplaser weisen wegen ihrer kurzen Resonatorlänge in der Regel eine weitgehend glatte Pulsform auf, ihre Maximalenergie ist aber auf Energien im Mikrojoulebereich beschränkt. Ist man an gaußförmigen Pulsen mit größeren Ausgangsenergien interessiert, benutzt man einen single-longitudinal-mode Laseroszillator als "Seeder" für einen weiteren Oszillator mit hoher Ausgangsleistung, in dem durch die starke Verstärkung des Seed-Mode das Anschwingen anderer Longitudinalmoden unterdrückt wird. Hierfür muss allerdings die Resonatorlänge *L* des Verstärkungs-Oszillators an die Wellenlänge des Seeders angepaßt werden, um die Resonatorumlaufbedingung *L* = n *λ*/2 zu erfüllen, was die Komplexität des Gesamtsystems beträchtlich erhöht.

Tatsächlich liefern Gaußpulse aus einem longitudinal einmodigen Laserresonator eine wesentlich schärfere Schwelle für den optischen Durchbruch als reguläre Pulse, wie der Vergleich in Fig. 2 offenbart. Die Schwellenschärfe S = Eₜₕ/ΔE, mit ΔE = Energiebereich zwischen 10% und 90% Durchbruchswahrscheinlichkeit hat für reguläre Nd:YAG Nanosekundenpulse (t_{L} = 11.2 ns, λ = 1064 nm, NA = 0.8) den Wert S = 2,7, für gaußförmige Nanosekundenpulse (t_{L} = 11.2 ns, λ = 1064 nm, NA = 0.8) den Wert S = 25 und für Femtosekundenpulse (t_{L} = 315 fs, λ = 1040 nm, NA = 0.8) den Wert S = 31. Nanosekundenpulse mit zeitlich glattem Strahlprofil erzeugen also auf ähnlich reproduzierbare und vorhersagbare Weise einen optischen Durchbruch wie Femtosekundenpulse.

Dieser deterministische Charakter des optischen Durchbruchs mit Nanosekundenpulsen kann durch Verunreinigungen im Zielmaterial beeinträchtigt werden, welche durch lineare Absorption und thermische Ionisierung zur Erzeugung von Startelektronen für die Lawinenionisation führen. Bei Verwendung großer numerischen Aperturen muss allerdings die Verunreinigungsdichte sehr hoch sein, um eine solche Auswirkung zu haben. So würde sich z.B. bei NA = 0.8 (NA - numerische Apertur) und einer Verunreinigungsdichte von 10¹⁰ cm⁻³ nur mit 1% Wahrscheinlichkeit eine Verunreinigung im Fokusvolumen befinden. Solch starke Verunreinigungen treten in der Praxis sehr selten auf, und daher kann bei Verwendung zeitlich glatter ns- Laserpulse für eine große Klasse von Fällen von einem deterministischen Zusammenhang zwischen Pulsenergie und Durchbruchschwelle ausgehen.

Es ist deshalb erfindungsgemäß vorgesehen, zur Materialbearbeitung Laserpulse mit glattem zeitlichem Strahlprofil einzusetzen. Die Erzeugung von Nano- und Mikroeffekten setzt zwar die Verwendung zeitlich glatter Laserpulse voraus, nicht aber deren Erzeugung in einem geseedeten Lasersystem. Mikrochiplaser mit sehr kurzem Resonator emittieren ebenfalls zeitlich glatte Pulse, die sich für die Erzeugung der Nanoeffekte eignen.

Bei der Untersuchung des optischen Durchbruchs in Wasser mit Laserpulsen aus dem UV-, VIS- und IR-Spektrum (Nd:YAG, 1064 nm und 2. und 3. Harmonische) ergaben sich u. a. die in Fig. 3 gezeigten Messwerte. Die gemessenen Blasengrößen sind gegenüber der im Fokus eingestrahlten Pulsenergie aufgetragen. Besonders hervorzuheben sind die Messwerte im rechten unteren Bildbereich (E < 0.1 µJ, Blasenradius < 10 µm). Sie markieren das Aufschwingen messbarer - und für die Bearbeitung etwa von Zellen wirksamer - Blasen ohne Auftreten von Plasmaleuchten, deren Größe mit wachsender Pulsenergie ansteigt.

Mittels glatter Nanosekundenpulse lassen sich ebenso wie durch Femtosekundenpulse Blasen mit weniger als 1 µm Maximalradius erzeugen. Das für die Erzeugung von Blasengrößen < 5 µm verfügbare Energieintervall wird mit ansteigender Laserpulsdauer immer breiter. Bei Erhöhung der Laserpulsenergie wird schließlich die Schwelle für die Bildung leuchtender Plasmen überschritten, was die Obergrenze des erfindungsgemäßen Arbeitsbereichs definiert.

Das Plasmaleuchten setzt an einer scharf messbaren Plasmaleuchtschwelle ein. Das Auftreten des Plasmaleuchtens geht mit einer abrupten Zunahme der Plasmagröße, der Größe der erzeugten Blasen und des Umwandlungsgrades von Laserenergie in die mechanische Energie der Blasen einher. In Fig. 3 ist dieser Übergang jeweils durch Sprünge im Wert des Blasenradius gekennzeichnet. Oberhalb der Schwelle für das Plasmaleuchten sinkt daher die räumliche Lokalisierbarkeit und Präzision der Lasereffekte drastisch ab.

Mit Femtosekundenpulsen lassen sich ebenfalls Nano- und Mikroeffekte erzeugen, es existiert aber kein separater Bereich kleiner Blasen mit relativ langsamem Anstieg des Blasenradius, wie er mit Pulsdauern von 0.9 bis 11 ns beobachtet wird. Ein separater Bereich kleiner Blasen beginnt sich bei Pulsdauern ab etwa 300 ps auszubilden und seine Breite (im Bereich der Laserpulsenergien) nimmt mit wachsender Pulsdauer zu.

Die im Sinne der vorliegenden Erfindung zu detektierende Bearbeitungsschwelle ist beim hier diskutierten Beispiel die Schwelle für die Blasenbildung, in Fig. 3 gekennzeichnet durch den jeweiligen linken Endpunkt einer Messpunkt-Reihe.

Sie entspricht der Schwelle für den Phasenübergang in Wasser und somit der Schwelle für Materialabtrag durch Einzelpulse in wasserhaltigen Materialien wie z. B. biologischem Gewebe oder Zellen. Auch oberhalb der Blasenbildungsschwelle bleiben die Blasen zunächst noch sehr klein, d.h. der Lasereffekt weist eine hohe räumliche Präzision auf.

Für Wasser liegt die Bearbeitungsschwelle - ausgedrückt als Bestrahlungsstärke ("Irradiance") - für die verwendeten Wellenlängen 355 nm und 532 nm und Pulsdauern 0,9 bis 11 ns zwischen 90 GW/cm² und 170 GW/cm². Die Plasmaleuchtschwellen für die helle Plasmaluminiszenz und große Blasen liegen je nach Pulsdauer und Wellenlänge um einen Faktor 1,5 (bei 1 ns, 532 nm) bis 25 (bei 1 ns, 355 nm) höher.

Der Energiebereich, in dem sich Nano- und Mikroeffekte mit Pulsen zwischen 0,9 und 11 ns Pulsdauer erzeugen lassen, ist groß und weist scharfe Grenzen auf, und die Effektgrößen sind sehr gut reproduzierbar. Es ist also möglich, zuverlässig und ohne statistische Ausreißer Nano- und Mikroeffekte zu erzeugen.

Für praktische Anwendungen sollte man vorzugsweise einen konkreten Arbeitspunkt definieren, etwa durch jene Bestrahlungsstärke, die erstmals eine vorab definierte, für den Bearbeitungszweck wirksame Blasengröße erzeugt. Der Arbeitspunkt sollte dabei einigen Abstand zur Plasmaleuchtschwelle aufweisen, um das Einsetzen des Plasmaleuchtens sicher zu vermeiden. Dass sich ein solcher Arbeitspunkt überhaupt zuverlässig finden lässt, war bislang nicht bekannt.

In der Praxis wird die Bestrahlungsstärke nicht nur durch die Laserparameter bestimmt, sondern auch durch den Lichtübertragungsweg bis zum Zielort und die Fokussierungsbedingungen beeinflusst. Es empfiehlt sich daher, vor dem Einsatz des Verfahrens Bearbeitungsschwelle und Plasmaleuchtschwelle für die konkrete Bearbeitungsaufgabe zu bestimmen und eventuell zusätzlich Kennlinien für den Zusammenhang zwischen Pulsenergie und Effektstärke aufzunehmen.

Hierzu eignen sich bei Festkörpern mikroskopische Inspektion des Fokusbereichs oder statische Streulichtverfahren (in Transmission, unter 90° oder in Reflexion), bei Flüssigkeiten oder Gewebe müssen dynamische Streulichtverfahren zum Einsatz kommen. Eine mögliche Realisierung hiervon wird im Stand der Technik als Blasengrößenmessung zitiert. Streulichtverfahren eignen sich auch zur Online-Kontrolle der Nano- und Mikrobearbeitung und als Sicherheitssystem bei klinischen oder kosmetischen Gewebebehandlung, bei der das Auftreten von leuchtenden Plasmen sofort zur Unterbrechung der Behandlung führen sollte. Die Möglichkeit der Erzeugung von Nano-und Mikroeffekten ohne Plasmaleuchten wurde für numerische Aperturen im Bereich 0.25 < NA < 0.9 nachgewiesen, ist aber wahrscheinlich auch bei noch größeren oder kleineren Aperturen möglich.

Es sollten Wellenlängen im Bereich von 300 nm bis 1000 nm verwendet werden. Das Laserlicht muss in das Zielmaterial eindringen können. Das erfordert bei biologischem Gewebe und Glas Wellenlängen größer als 300 nm. Bei 1064 nm wird kein Bereich mit Nano- und Mikroblasen mehr festgestellt.. Hieraus ergibt sich der genannte Bereich.

Der theoretische Hintergrund des Auftretens separierter Schwellwerte und insbesondere die gefundene Limitierung auf Wellenlängen unter etwa 1000 nm sind mit Hilfe von Modellberechnungen nachvollziehbar. Deren Ergebnisse sollen nachfolgend kurz zusammengefasst werden:
Die Zweistufigkeit des optischen Durchbruch-Prozesses lässt sich durch Modellierung der Plasmabildung unter Einbeziehung der Multiphotonenionisation, Lawinenionisation, Diffusionsverlusten, Rekombination und thermischer Ionisierung erklären. Eine solche Modellierung wird im Rahmen der hier beschriebenen Untersuchung erstmals durchgeführt; frühere Modelle berücksichtigten in der Regel Multiphotonenionisation, Lawinenionisation und Diffusionsverluste; die Rekombination wird meist erwähnt aber in den Rechnungen vernachlässigt, und die thermische Ionisierung wurde bei der Modellierung des optischen Durchbruchs in transparenten Medien zuvor noch gar nicht berücksichtigt.

Die Rechnungen ergeben, dass mit ansteigender Laserintensität der Ionisierungsgrad zunächst rasch ansteigt, dann aber auf einem Plateau annähernd stagniert. In diesem Bereich wird die Ionisierungslawine (proportional zur freien Elektronendichte *ρ* und zur Laserintensität) durch die Rekombination (proportional zu *ρ*²) gebremst, so dass ein low-density Plasma vorliegt. Während des Laserpulses werden durch Multiphotonen- und Lawinenionisation ständig neue freie Elektronen erzeugt, die bei Rekombination ihre Energie abgeben und das Medium aufheizen. Sobald die bis zum Ende des Laserpulses deponierte räumliche Energiedichte die für einen Phasenübergang erforderliche Energiedichte übersteigt, wird eine Blase (in Wasser) oder eine Kavität (in Festkörpern) erzeugt.

Bei noch weiter anwachsender Laserintensität steigen Energiedichte und Temperatur im Laserfokus an, bis schließlich thermische Ionisierung in nennenswertem Umfang auftreten kann. Wenn die thermische Ionisierung so stark wird, dass sie zusammen mit der Lawinenionisation die bremsende Wirkung durch Rekombinationseffekte überwindet, steigt der Ionisierungsgrad sehr schnell an, bis schließlich volle Ionisierung erreicht ist. Dieser Anstieg am oberen Ende des low-density Plasmabereichs führt zur Entstehung von high-density Plasma und entspricht dem Auftreten des intensiven Plasmaleuchtens, das üblicherweise mit dem optischen Durchbruch durch Nanosekundenpulse identifiziert wird.

Ein zeitweiliges Gleichgewicht zwischen Lawinenionisation und Rekombinationsvorgängen während des Laserpulses setzt voraus, dass die Laserpulsdauer deutlich länger ist als die Zeitkonstante der Rekombination. Man kann davon ausgehen, dass diese Zeitkonstante für die meisten transparenten Materialien durch etwa 100 ps gut nach oben abgeschätzt ist. Folglich ist das Auftreten des Gleichgewichts erst bei einigen hundert Pikosekunden Pulsdauer möglich. Die experimentellen Befunde legen eine untere Grenze von 300 ps nahe.

Bei kürzeren Pulsdauern gibt es einen fließenden Übergang zwischen dem Auftreten von Blasenbildung und der Entstehung des Plasmaleuchtens bei größeren Laserintenisitäten bzw. Pulsenergien. Bei längeren Pulsdauern gibt es einen separaten low-density Plasmabereich mit niedriger räumlicher Energiedichte und besonders kleinen Blasen und danach einen abrupten Anstieg des Ionisierungsgrades, der Energiedichte und der Blasengröße.

In transparenten Medien müssen für die Initiierung des optischen Durchbruchs generell zunächst durch Multiphotonenionisation Startelektronen für die Ionisierungslawine gebildet werden. Bei λ = 1064 nm ist dies die entscheidende Hürde für die Plasmabildung, da wegen der geringen Photonenenergie ein Multiphotonenprozess hoher Ordnung erforderlich ist. Beispielsweise wird zur Überwindung der Bandlücke in Wasser die Energie von 6 simultan absorbierten Photonen benötigt. Deshalb ist für die Erzeugung der Startelektronen eine sehr hohe Intensität erforderlich. Diese Intensität treibt die nachfolgende Ionisierungslawine zu sehr hohen freien Elektronendichten, deren weiterer Anstieg nur für Bruchteile der Laserpulsdauer durch Rekombinationsprozesse aufgehalten werden kann. Danach ist die Temperatur im Fokusvolumen auf so hohe Werte angestiegen, dass thermische Ionisierung und Lawinenionisation gemeinsam den Durchbruchsprozess bis zur vollen Ionisierung weiter treiben. Bei λ = 1064 nm führt der optische Durchbruch daher bereits an der Blasenbildungsschwelle zu sehr hohen Energiedichten weit oberhalb des für einen Phasenübergang erforderlichen Wertes und es existiert kein separater Energiebereich, in dem Mikroeffekte erzeugt werden können.

Diese Analyse lässt sich auf alle transparenten Medien mit Energie-Bänderstruktur anwenden. Daraus folgt aber, dass das erfindungsgemäße Verfahren allgemeingültigen Charakter hat und die Möglichkeit der Erzeugung von Nano- und Mikroeffekten nicht auf Wasser oder wässrige Medien beschränkt ist.

Die Rechnungen zeigen für UV/VIS Nanosekundenpulse insbesondere im Bereich des Phasenübergangs (also z.B. Blasenbildung) einen allmählichen Anstieg des Ionisierungsgrades mit der Laserintensität. Dies bedeutet, dass es auch unterhalb der Schwelle für den Phasenübergang eine große Zahl freier Elektronen gibt und man die Elektronendichte durch Variation der Laserintensität einstellen kann. Man kann daher auch mit Nanosekundenpulsen über freie Elektronen vermittelte chemische oder thermische Effekte ohne gleichzeitigen Phasenübergang erzeugen, wie von Vogel et al. in Appl. Phys. B 81:1015-1047 (2005) für Femtosekundenpulse beschrieben. Erfindungsgemäß ist für diese Zielsetzung eine Bearbeitungsschwelle unterhalb der des Phasenübergangs zu definieren, die sich nunmehr durch das Einsetzen messbarer Materialveränderungen, beispielsweise eine Änderung des Brechungsindex oder Strukturumwandlungen infolge der Anwesenheit freier Elektronen, auszeichnet. Dies lässt sich z.B. zum Schreiben von Wellenleitern mit UV/VIS Nanosekundenpulsen ausnutzen.

Zur Abgrenzung von der Patentschrift DE 198 55 623 C1 sei an dieser Stelle betont, dass mit dem erfindungsgemäßen Verfahren eine Energiedeponierung auch in völlig transparente Medien ohne lineare Absorption mit Nanosekundenpulsen möglich ist. Die dort verwendete Pulsdauer 100 ns ist überdies höchstwahrscheinlich für das hier beschriebene Verfahren nicht geeignet, da solch lange Pulse sicher kein glattes zeitliches Strahlprofil aufweisen. Bereits einzelne Energiespitzen im Pulsverlauf können das Plasmaleuchten auslösen und würden dadurch die Lokalisierung des Bearbeitungseffektes zerstören. Vorzugsweise sollten Pulslängen bis maximal 20 ns verwendet werden.

Weiterhin ist auch ein Laser vorgesehen, der sich insbesondere zur Laserbehandlung von Augenkrankheiten eignet. Der erfindungsgemäße Laser, beispielsweise ein Festkörper- oder Mikrochip-Laser, ist zur Bearbeitung eines transparenten Materials, beispielsweise der Hornhaut des Auges, durch im Bereich des Laserfokus erfolgende nichtlineare Absorption gepulster Laserstrahlung eingerichtet und weist eine Wellenlänge in einem Bereich von 300 bis 1000 nm, eine Pulslänge in einem Bereich von 300 ps bis 20 ns und ein Mittel zum Einstellen der Bestrahlungsstärke auf, wobei der Laser Laserpulse mit einem zeitlich glatten Strahlprofil erzeugt.

Erfindungsgemäß wird der Bearbeitungserfolg des Lasers durch den Bearbeitungserfolg registrierende, auf das Mittel zum Einstellen der Bestrahlungsstärke wirkende Inspektionsmittel überprüft, die derart auf das Mittel wirken, dass die Bestrahlungsstärke innerhalb eines Intervalls zwischen der Nachweisgrenze der Inspektion und dem Auftreten von Plasmaleuchten im bearbeiteten Material liegt.

Bevorzugt können die Inspektionsmittel während der Bearbeitung im bearbeiteten Material erfolgende Blasenbildung registrieren, sodass die Nachweisgrenze der Inspektion durch die Registrierung einer ersten Blasenbildung definiert ist. Die obere Grenze des Intervalls wird durch das Auftreten von Plasmaleuchten gebildet, wobei dieser Zustand jedoch nicht eintreten wird, da die Mittel zum Einstellen der Bestrahlungsstärke darauf hinwirken, dass die Bestrahlungsstärke innerhalb dieser Intervallgrenzen liegt. So können irreparable Schädigungen des Auges verhindert werden.

Abschließend sollen noch die Vorteile der Erfindung hervorgehoben werden:
Die räumliche Lokalisierbarkeit ist besser als mit ultrakurzen Laserpulsdauern, weil nichtlineare Ausbreitungseffekte vernachlässigbar sind. An der Bearbeitungsschwelle ist die Spitzenleistung in einem UV-Nanosekundenpuls um zwei Größenordnungen niedriger als bei einem UV-Femtosekundenpuls.

Lokalisierte Materialbearbeitung an Zielorten tief innerhalb von transparenten Materialien kann mit dem erfindungsgemäßen Verfahren viel einfacher durchgeführt werden als mit Femtosekundenpulsen, denn hierfür müssen in der Regel Optiken mit großem Arbeitabstand und daher meist relativ kleiner numerischer Apertur eingesetzt werden. In diesem Fall treten bei Verwendung von Femtosekundenpulsen wegen der höheren Spitzenleistungen leichter nichtlineare Ausbreitungseffekte und Filamentierung auf als mit dem erfindungsgemäßen Verfahren.

Der nutzbare Energiebereich ist größer als bei ultrakurzen Laserpulsdauern (vgl. Fig. 3). Nanosekundenlaser (vor allem Mikrochiplaser) sind viel kostengünstiger und kompakter als Ultrakurzpulslaser.

## Patentansprüche

1. Verfahren zur Bearbeitung eines transparenten Materials durch im Bereich eines Laserfokus erfolgende nichtlineare Absorption gepulster Laserstrahlung, mit den Schritten
- Auswählen einer Laserwellenlänge aus dem Intervall von 300 bis 1000 nm,
- Auswählen einer Pulslänge aus dem Intervall von 300 ps bis 20 ns,
- Applizieren von Laserpulsen mit zeitlich glattem Strahlprofil,
**dadurch gekennzeichnet, dass**
die Bestrahlungsstärke aus einem für das zu bearbeitende Material vorbestimmten Intervall ausgewählt ist, in dem Plasmabildung ohne Plasmaleuchten auftritt.

2. Verfahren nach Anspruch 1, **dadurch gekennzeichnet, dass** das für die Bestrahlungsstärke vorbestimmte Intervall in Abhängigkeit von dem zu bearbeitenden Material unter Variation der Bestrahlungsstärke im Laserfokus durch Inspektion des Bearbeitungserfolges ermittelt ist, wobei die untere Intervallgrenze der Bestrahlungsstärke anhand der Nachweisgrenze der Inspektion und die obere Intervallgrenze anhand des Auftretens von Plasmaleuchten bestimmt ist.

3. Verfahren nach einem der vorangehenden Ansprüche, **dadurch gekennzeichnet, dass** das transparente Material ein flüssiges Medium ist und im flüssigen Medium Blasen mit einem fest gewählten Radius gebildet werden.

4. Verfahren nach Anspruch 3, **dadurch gekennzeichnet, dass** das transparente Material ein wässriges Medium ist und die Bestrahlungsstärke für das wässrige Medium wenigstens 90 GW/cm² beträgt.

5. Verfahren nach einem der Ansprüche 1 oder 2, **dadurch gekennzeichnet, dass** das transparente Material ein Festkörper ist und im Festkörper Hohlräume gebildet werden.

6. Verfahren nach einem der Ansprüche 1 oder 2, **dadurch gekennzeichnet, dass** das transparente Material ein Festkörper ist und der Brechungsindex im Festkörper lokal verändert wird.

7. Verfahren nach einem der vorangehenden Ansprüche, **dadurch gekennzeichnet, dass** die Pulsdauer wenigstens das Dreifache der Zeitkonstante der Plasmarekombination des zu bearbeitenden Materials beträgt.

8. Verfahren nach einem der vorangehenden Ansprüche, **dadurch gekennzeichnet, dass** die Pulsdauer aus dem Intervall von 0,9 bis 11 ns gewählt wird.

9. Vorrichtung mit einem Laser zur Bearbeitung eines transparenten Materials durch im Bereich des Laserfokus erfolgende nichtlineare Absorption gepulster Laserstrahlung, mit einer Wellenlänge in einem Bereich von 300 bis 1000 nm und einer Pulslänge in einem Bereich von 300 ps bis 20 ns und einem Mittel zum Einstellen der Bestrahlungsstärke, wobei der Laser Laserpulse mit einem zeitlich glatten Strahlprofil erzeugt,
**gekennzeichnet durch**
den Bearbeitungserfolg des Lasers registrierende, auf das Mittel zum Einstellen der Bestrahlungsstärke derart wirkende Inspektionsmittel, dass die Bestrahlungsstärke innerhalb eines Intervalls zwischen der Nachweisgrenze der Inspektion und dem Auftreten von Plasmaleuchten im bearbeiteten Material liegt.

10. Vorrichtung nach Anspruch 9, **dadurch gekennzeichnet, dass** die Inspektionsmittel während der Bearbeitung im bearbeiteten Material erfolgende Blasenbildung registrieren.

11. Vorrichtung nach einem der Ansprüche 9 und 10 oder zur Durchführung des Verfahrens nach einem der Ansprüche 1 bis 8, **dadurch gekennzeichnet, dass** der Laser ein geseedeter Festkörperlaser oder ein Mikrochip-Laser ist.

## Claims

1. Method for treating a transparent material by the non-linear absorption of pulsed laser radiation taking place in the region of a laser focus, comprising the steps
- selecting a laser wavelength from the interval from 300 to 1000 nm,
- selecting a pulse length from the interval from 300 ps to 20 ns,
- applying laser pulses with a temporally smooth beam profile,
**characterised in that**
the irradiance is selected from an interval predetermined for the material to be treated, in which plasma formation occurs without plasma luminescence.

2. Method according to Claim 1, **characterised in that** the interval predetermined for the irradiance is determined as a function of the material to be treated, by varying the irradiance in the laser focus by inspection of the treating success, wherein the lower interval limit of the irradiance is determined using the detection limit of the inspection and the upper interval limit is determined using the occurrence of plasma luminescence.

3. Method according to one of the preceding claims, **characterised in that** the transparent material is a liquid medium and bubbles with a fixedly selected radius are formed in the liquid medium.

4. Method according to Claim 3, **characterised in that** the transparent material is an aqueous medium and the irradiance for the aqueous medium is at least 90 GW/cm².

5. Method according to one of Claims 1 or 2, **characterised in that** the transparent material is a solid and cavities are formed in the solid.

6. Method according to one of Claims 1 or 2, **characterised in that** the transparent material is a solid and the refractive index in the solid is changed locally.

7. Method according to one of the preceding claims, **characterised in that** the pulse duration is at least three times the time constant of the plasma recombination of the material to be treated.

8. Method according to one of the preceding claims, **characterised in that** the pulse duration is selected from the interval from 0.9 to 11 ns.

9. Apparatus comprising a laser for treating a transparent material by non-linear absorption of pulsed laser radiation taking place in the region of the laser focus, having a wavelength in a range from 300 to 1000 nm and a pulse length in a range from 300 ps to 20 ns, and a means for setting the irradiance, wherein the laser produces laser pulses with a temporally smooth beam profile,
**characterised by**
inspection means which register the treating success of the laser and act on the means for setting the irradiance, such that the irradiance is within an interval between the detection limit of the inspection and the occurrence of plasma luminescence in the treated material.

10. Apparatus according to Claim 9, **characterised in that** the inspection means register bubble formation taking place during the machining in the treated material.

11. Apparatus according to one of Claims 9 and 10, or for carrying out the method according to one of Claims 1 to 8, **characterised in that** the laser is a seeded solid-state laser or a microchip laser.

## Revendications

1. Procédé d'usinage d'un matériau transparent par l'absorption non linéaire, au niveau d'un foyer laser, d'un rayon laser pulsé, avec les étapes suivantes :
- sélection d'une longueur d'onde laser dans l'intervalle de 300 à 1000 nm,
- sélection d'une longueur d'impulsion dans l'intervalle de 300 ps à 20 ns,
- application d'impulsions laser avec un profil de rayonnement temporellement lisse,
**caractérisé en ce que**
l'intensité de l'irradiation est sélectionnée dans un intervalle prédéterminé pour le matériau à usiner, dans lequel survient la formation d'un plasma sans luminescence du plasma.

2. Procédé selon la revendication 1, **caractérisé en ce que** l'intervalle prédéterminé pour l'intensité de l'irradiation est déterminé en fonction du matériau à usiner en faisant varier l'intensité d'irradiation dans le foyer laser par inspection du résultat de l'usinage, la limite inférieure de l'intervalle de l'intensité d'irradiation étant déterminée à l'aide de la limite de détection de l'inspection et la limite supérieure de l'intervalle étant déterminée à l'aide de l'apparition d'une luminescence du plasma.

3. Procédé selon l'une des revendications précédentes, **caractérisé en ce que** le matériau transparent est un milieu liquide et dans le milieu liquide des bulles sont formées avec un rayon fixé.

4. Procédé selon la revendication 3, **caractérisé en ce que** le matériau transparent est milieu aqueux et l'intensité d'irradiation pour le milieu aqueux est égale à au moins 90 GW/cm².

5. Procédé selon l'une des revendications 1 ou 2, **caractérisé en ce que** le matériau transparent est corps solide et dans le corps solide des espaces creux sont formés.

6. Procédé selon l'une des revendications 1 ou 2, **caractérisé en ce que** le matériau transparent est un corps solide et l'indice de réfraction du corps solide est modifié localement.

7. Procédé selon l'une des revendications précédentes, **caractérisé en ce que** la durée des impulsions est égale à au moins le triple de la constante de temps de la recombinaison du plasma du matériau à usiner.

8. Procédé selon l'une des revendications précédentes, **caractérisé en ce que** la durée des impulsions est choisie dans l'intervalle entre 0,9 et 11 ns.

9. Dispositif avec un laser pour l'usinage d'un matériau transparent par l'absorption non linéaire, au niveau du foyer laser, d'un rayon laser pulsé, avec une longueur d'onde de l'ordre de 300 à 1000 nm et une longueur d'impulsions de l'ordre de 300 ps à 20 ns et un moyen de réglage de l'intensité d'irradiation, le laser générant des impulsions laser avec un profil de rayonnement temporellement lisse,
**caractérisé par**
des moyens d'inspection enregistrant le résultat de l'usinage, agissant sur le moyen de réglage de l'intensité d'irradiation de façon à ce que l'intensité d'irradiation se trouve dans un intervalle entre la limite de détection de l'inspection et l'apparition d'une luminescence plasma dans le matériau traité.

10. Dispositif selon la revendication 9, **caractérisé en ce que** les moyens d'inspection enregistrent la formation de bulles dans le matériau usiné pendant l'usinage.

11. Dispositif selon l'une des revendications 9 et 10, pour la réalisation du procédé selon l'une des revendications 1 à 8, **caractérisé en ce que** le laser est un laser solide amorcé ou un laser à micro-puce.
